(19) Europäisches Patentamt
European Patent Office
Office européen des brevets

(11) Publication number: **0 282 771 B1**

(12) **EUROPEAN PATENT SPECIFICATION**

(45) Date of publication of patent specification: **06.05.92**

(51) Int. Cl.5: **C08G 18/48**, C08G 18/75, C08G 18/10, C08G 18/32, A61F 13/02, A61L 15/22

(21) Application number: **88102626.4**

(22) Date of filing: **23.02.88**

(54) **A nonporous film of polyurethane resin.**

(30) Priority: **24.02.87 JP 40967/87**

(43) Date of publication of application:
**21.09.88 Bulletin 88/38**

(45) Publication of the grant of the patent:
**06.05.92 Bulletin 92/19**

(84) Designated Contracting States:
**DE FR GB**

(56) References cited:
**EP-A- 0 050 035**
**EP-A- 0 091 800**
**EP-A- 0 206 217**
**DE-A- 2 157 203**
**US-A- 3 425 999**
**US-A- 3 849 360**

(73) Proprietor: **MITSUBISHI KASEI CORPORATION**
**5-2, Marunouchi 2-chome Chiyoda-ku**
**Tokyo 100(JP)**

(72) Inventor: **Tsuge, Yukio**
**4080-83, Kokubu**
**Ebina-shi Kanagawa(JP)**
Inventor: **Kobayashi, Tatsuhiko**
**Popuragaoka Kopu 21-202, 2-32-4, Narasedai**
**Machida-shi Tokyo(JP)**

(74) Representative: **Vossius & Partner**
**Siebertstrasse 4 P.O. Box 86 07 67**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 282 771 B1

## Description

This invention relates to a nonporous film of a polyurethane resin having an excellent permeability and, particularly, being useful as a medical film. More particularly it relates to a nonporous film of a polyurethane resin which is nonporous and highly permeable and shows no yellowing when exposed to, for example, light or $NO_x$ gas and slightly swells when absorbing water.

It is well-known that the permeability of a polyurethane resin can be improved by incorporating polyethylene glycol to the polyol component thereof [cf. J. Appl. Polymer Sci., 16, 2105 - 2114 (1972)]. However this process is accompanied by some disadvantages such that an increase in the polyethylene content in the polyol component would exert not only the desired effect of improving the permeability of the polyurethane resin but also undesirable ones of significantly promoting the swelling on water absorption and lowering the mechanical properties thereof.

It is well known that a highly permeable waterproof fabric can be obtained by making a polyurethane film porous through wet film formation. However nonporous film materials are frequently preferred when a film should have a high permeability or strength or for medical or sanitation materials which should not be contaminated with bacteria or filth. It has been difficult to obtain a film suitable therefor, which is highly permeable, slightly swells when absorbing water and has well-balanced physical properties from a practical viewpoint.

It is an object of the present invention to provide a nonporous film of a polyurethane resin which is nonporous and highly permeable and shows a slight dimensional change caused by swelling on water absorption and no yellowing when exposed to, for example, light or $NO_x$ gas.

It is another object of the present invention to provide a film material for medical uses which has a nonporous structure without showing any holes, pores or discontinuous sections and exhibits excellent physical properties including a sufficient permeability as well as such a high flexibility as to never lower the adhesiveness thereof when applied onto a bending or curved part, for example, a human joint.

The above object of the present invention can be achieved by providing a nonporous film of a polyurethane resin obtained by reacting a random copolymer of tetrahydrofuran and ethylene oxide, which contains 20 to 80% by weight of ethylene oxide units and has a number-average molecular weight of 800 to 3,000, with alicyclic isocyanate(s); and then lengthening the chain of the resulting product with alicyclic diamine(s).

According to the present invention, there is to further provide a medical film which comprises a nonporous film of a polyurethane resin obtained by reacting a random copolymer of tetrahydrofuran and ethylene oxide, which contains 20 to 80% by weight of ethylene oxide units and has a number- average molecular weight of 800 to 3,000, with an alicyclic diisocyanate, and lengthening a chain of the obtained product with an alicyclic diamine.

The polyurethane resin of the present invention essentially comprises a random copolymer of tetrahydrofuran, which will be abbreviated as THF hereinafter, and ethylene oxide, which will be abbreviated as EO hereinafter, as a polyol component. This random copolymer may be synthesized by subjecting a mixture of THF and EO to ring opening copolymerization with the use of water or short chain diol(s) such as ethylene glycol or 1,4-butanediol as an initiator in the presence of a Lewis acid catalyst such as boron trifluoride ether complex salt.

In order to inhibit the swelling of the aimed resin on water absorption and to minimize lowering of physical physical properties thereof, this THF/EO random copolymer should contain 20 to 80 % by weight, preferably 30 to 70 % by weight, of EO units. The number-average molecular weight of the THF/EO random copolymer is 800 to 3000. A random copolymer having a number-average molecular weight smaller than 800 would give a hard polyurethane film, while a number-average molecular weight exceeding 3000 would give a polyurethane film which is highly sticky and considerably swells on water absorption. A number-average molecular weight within a range of 1000 to 2500 is preferable in order to achieve the optimum physical properties of the film.

Polytetramethylene ether glycol, which will be abbreviated as PTMG hereinafter, may be added to the THF/EO random copolymer, if required. In this case, it is desirable to use PTMG having an average molecular weight of 800 to 3000; and to control the number-average molecular weight of the resulting polyol mixture within 800 to 3000, preferably 1000 to 2500, in order to give a polyurethane film of well-balanced physical properties.

The abovementioned THF/EO random copolymer may include a partial block copolymerized structure formed during the polymerization in some polyol chains, without departing from the spirit or scope of the present invention.

In addition to the abovementioned method, the THF/EO copolymer may be obtained by adding EO to PTMG ring opening-polymerized with THF; or by adding THF to polyethylene glycol, which will be abbreviated with PEG hereinafter, ring opening-copolymerized with EO. However polyurethane resins obtained with the use of these block copolymers would significanlty swell with an increase in the EO content in the total polyurethane, similar to the case of a mixture of PTMG and PEG, since these copolymers include highly hydrophilic EO homopolymer long-chains therein. Thus these block copolymers are undesirable in practice.

We have found that a polyurethane resin comprising the abovementioned THF/EO random copolymer as the polyol component shows a surprisingly high permeability, though an increase in the EO units therein is accompanied by only limited increase in the water absorption ratio, compared with a THF/EO block copolymer or PTMG/PEG system.

The aimed polyurethane resin of the present invention may be produced by reacting the polyol as specified above with an excessive amont of alicyclic diisocyanate(s) at 70 to 120 °C to give an urethane polymer having a terminal isocyanate group and then lengthening the chain of the resulting polymer with the use of alicyclic diamine(s) in an organic solvent at 20 to 40 °C.

The equivalent ratio of the alicyclic diisocyanate(s) and polyol in the urethane polymer is usually within 1.5 to 4 : 1, although an equivalent ratio thereof within 1.8 to 3.2 : 1 is preferable in order to give the final product having excellent physical properties as well as a high permeability.

Examples of the alicyclic diisocyanate to be used in the present invention include isophorone diisocyanate, 4,4′-dicyclohexylmethane diisocyanate, 1,4-cyclohexylene diisocyanate and hydrogenated tolylene diisocyanate. Usually one of these diisocyanates is employed alone, although a mixture thereof may be employed. Among these compounds, 4,4′-dicyclohexylmethane diisocyanate is the most preferable since a film having well-balanced mechanical properties and permeability can be obtained with the use of the same.

It is not preferable to replace the alicyclic diisocyanate with an aliphatic one such as hexamethylene diisocyanate, since the resulting resin would show no yellowing but have poor mechanical properties and a low heat resistance. It is also unpreferable to replace the alicyclic diisocyanate with an aromatic one, since the resulting resin would have excellent physical properties but show serious yellowing when exposed to, for example, light or $NO_x$ gas.

Examples of the alicyclic diamine to be used in the present invention as a chain lengthening agent include isophoronediamine, 4,4′-dicyclohexyldiamine, 3,3′-dimethyl4,4′-dicyclohexylamine and 1,4-cyclohexylenediamine. Either one of these diamines or a mixture thereof may be employed.

The abovementioned alicyclic diamine is suitable for achieving a high stability of the polyurethane solution and excellent film properties. When it is replaced with an aliphatic diamine such as ethylenediamine, the resulting polyurethane solution has a poor stability and sometimes sets to gel upon storage. Further an aromatic diamine is not preferable since it is colored when oxidized. Isophoronediamine is particularly preferable from the viewpoint of the stability of the solution.

As the organic solvent to be used in the synthesis of the polyurethane in the present invention, those having a high solvent power, for example, dimethylformamide, dimethylacetamide and dimethylsulfoxide are suitable. One of these solvents may be employed alone. Alternatively it may be combined with one or more other solvents selected from among aromatic ones such as toluene and xylene; ketones such as methyl ethyl ketone, acetone and cyclohexanone; acetates such as ethyl acetate and butyl acetate; chlorinated ones such as dichloroethane; ethers such as tetrahydrofuran and dioxane; and alcohols such as methanol or isopropanol.

The polyurethane resin thus obtained preferably contains 15 to 60 % by weight, based on the total polyurethane, of ethylene oxide units. When the ethylene oxide unit content is less than 15 % by weight, the obtained film has an unsatisfactory permeability. On the other hand, when it exceeds 60% by weight, the obtained film shows a serious dimensional change or lowered physical properties at water absorption, which makes it not desirable.

In the present invention, a catalyst, which is conventionally used in the synthesis of urethane resins, for example, a tertiary amine such as triethylenediamine or an organic tin compound such as dibutyltin dilaurate may be employed to thereby accelerate the reaction, if required.

When the polyurethane resin is to be applied to, for example, a permeable waterproof fabric, one or more additives selected from among hindered phenol antioxidants, benzophenone or benzotriazole UV absorbers and hindered amine UV stabilizers may be further added thereto to improve the mechanical durability of the product. In this case, each additive may be added in an amount of 0.05 to 3 % by weight, based on the solid polyurethane. In order to achieve an excellent effect in a small amont, it may be preferably added in an amount of 0.2 to 1 % by weight. A hindered amine UV stabilizer exerts, in particular,

a remarkable effect on the diisocyanate polyurethane of the present invention. Thus it can significantly prevent the physical properties of the resin from lowering upon photodeterioration or hydrolysis even when employed alone.

The polyurethane resin of the present invention may further comprise other additives such as inorganic fillers or colorants, if desired.

Now film-formation processes will be described. It is well known that a porous polyurethane film has an improved permeability and conventional processes for preparing thereof include the follwing.

(1) A wet film-formation process wherein a polyurethane resin solution is applied onto a substrate and the solvent and other soluble matters therein are extracted on a coagulating bath.

(2) A process for preparing a porous film wherein a water-in-oil type emulsion of a polyurethane resin is applied on a substrate and then dried thereon by heating. However a porous film obtained by each of these processes has only a limited strength, elongation and durability when used alone. Thus it should be combined with a substrate. Further these films are disadvantageous in that pores are liable to be blocked off by, for example, sweat or soil, which causes a gradual decrease in the permeability.

In the preparation of a porous film, furthermore, differences in size or distribution of pores would frequently cause a scatter of the permeability of the film. Thus the preparation should be carried out under strictly controlled conditions in order to reproduce constant properties of the film.

The polyurethane film of the present invention is a nonporous one and inferior to a porous one in the permeability. However a stable permeability of a nonporous polyurethane film can be reproduced by applying a polyurethane resin solution onto a substrate or a release sheet and drying the same by heating, i.e., dry film-formation. The film thus obtained has a satisfactory strength, elongation and durability from a practical viewpoint, even if used alone. Thus it is highly useful as a permeable material for, e.g., medical adhesive films or sanitation materials through which no foreign matter should pass.

The substrate to be used at the dry film-formation is not particularly restricted. For example, a polyethylene or polypropylene film, a release sheet coated with a fluorine or silicone releasing agent or a fabric may be employed therefor.

The permeability of the sheet material of the present invention increases with a decrease in the thickness thereof. Therefore it is preferbale to use a release sheet of a uniform thickness. The application method is not particularly restricted and either a knife coater or a roll coater may be used therefor. The drying temperature may be arbitrarily determined depending on the capacity of the drier, so long as it falls within a range wherein neither insufficeint drying nor rapid desolvation is observed. A drying temperature of 60 to 130° C is preferable.

The thickness of the film thus formed is usually 10 to 200 $\mu$m and preferably 10 to 100 $\mu$m. The film of the present invention is characterized in that its permeability does not so largely depend on the thickness as those of other urethane films do. When the thickness of the film is lower than 10 $\mu$m, pinholes are frequently formed at the application and the film is liable to be blocked together, thus showing a poor workability. When it exceeds 100 $\mu$m, on the other hand, a sufficient permeability can not be achieved.

The nonporous film of the present invention is highly permeable and shows a permeability of 1,000 $g/m^2 \cdot 24$ hr or above, preferably 1,500 $g/m^2 \cdot 24$ hr or above, as determined according to JIS Z 0208, at a thickness of 10 to 100 $\mu$m. When the permeability is lower than the range as defined above, an uncomfortable steamy feel is observed when applied on the skin. Further it has a high flexibility of 20 $kg/cm^2$ or above, preferably 30 $kg/cm^2$ or above, at 100 % modulus. When the flexibility is lower than 20 $kg/cm^2$, the adhesiveness of the film becomes excessively high, which frequently causes blocking of the film. When it exceeds 80 $kg/cm^2$, on the other hand, the flexibility and permeability would fall.

When the nonporous film of the present invention is used as a medical film or tape, an adhesive for the skin is applied on the surface of one side thereof. Conventional adhesives, for example, natural gum, butyl gum, butylene/butadiene copolymer, polyisobutylene copolymer, acrylic polymers or polyurethane adhesives may be employed therefor. Among these adhesives, the polyurethane adhesives are preferred. The adhesive is generally applied thereon to give a thickness of 10 to 50 $\mu$m.

In the THF/EO random copolymer to be used in the present invention as the polyol component, a continuous chain of EO units showing high water absorption properties is divided into relatively short portions with scattering THF units. This fact may contribute to the high permeability of the polyurethane resin brought about by the hydrophilic EO units, in spite of the low water absorption of the resin.

According to the present invention, a polyurethane resin, a nonporous film obtained from which has a high permeability, slightly swells when absorbing water and is highly resistant against light, $NO_x$ gas or hydrolysis, can be obtained. This resin is highly useful in forming permeable waterproof fabrics or medical or sanitation materials.

That is to say, since a film of urethane resin is permeable, regardless of being nonporous, the film exhibits an excellent property in prohibiting bacteria passing therethrough, allowing perspiration, and having elasticity. Thus, a medical film of the present invention may be effectively used without causing any disease on skin, such as a wet feeling, pruriginons or eruption, when the film is applied over human skin, and without any disadvantage such as being appart from the skin due to a flection, so as to obtain early recovery from the diseases.

In order to further illustrate the present invention, and not by way of limitation, the following Examples will be given.

Preparation Examples 1 to 4: THF/EO random copolymer

According to each composition as defined in Table 1, THF and EO are random copolymerized in an autoclave at 30 ° C under atmospheric pressure, with the use of ethylene glycol as an initiator and $BF_3$ ethyl ether complex salt as an acid catalyst. After the completion of the copolymerization, the acid catalyst in the product was neutralized with an alkali. Then the precipitate was filtered and further dehydrated by blowing a dry nitrogen stream thereto at 100 ° C.

The THF/EO random copolymers A, B, C and D thus obtained, which will be called polyols A to D hereinafter, were each in the form of a colorless and transparent liquid. The number-average molecular weight and EO content thereof are shown in Table 1.

The number-average molecular weight was determined by hydroxyl number determination while the EO content was calculated from the amount of the starting materials.

Examples 1 to 3

1. Preparation of polyurethane solution

The polyols A, B and C as shown in Table 1 were used. Each polyol was reacted with 4,4′-dicyclohexylmethane diisocyanate at a ratio as defined in Table 2 under dry nitrogen atmosphere in a flask at 100 ° C for six hours to thereby give an urethane prepolymer having a terminal ioscyanate group. Then the chain of the obtained prepolymer was lengthened with the use of isophorone diamine as a chain-lengthening agent at 30 ° C for two hours, while maintaining the temperature at 30 ° C. Thus a viscous polyurethane solution containing 25 % by weight of solid polyurethane was obtained. The viscosities of the polyurethane solutions obtained from the polyols A, B and C were 35,000 cps, 20,000 cps and 15,000 cps respectively.

2. Formation of polyurethane film

Each polyurethane solution as prepared above was poured onto a glass plate provided with spacers and spreaded thereon with a glass bar to give a uniform thickness. After drying at 80 ° C over day and night, a colorless and transparent dry polyurethane film was obtained. The spacers were located to adjust the thickness of the film to approximately 50µm. The film thus obtained was cured in a thermo-hygrostat at a temperature of 23 ° C under a relative humidity of 60 % prior to a performance evaluation test.

3. Performance evaluation test on polyurethane film

Dimensional increase at water-absorption:

A polyurethane film piece (50 mm × 50 mm) was immersed in water at 23 ° C for 24 hours and then the size was measured. Thus the dimensional increase was calculated from the dimensions before and after the water-absorption.

Permeability:

The permeability of the polyurethane film was determined by weighing the same with the use of a permeability cup at 40 ° C under a relative humidity of 90 %, according to JIS Z-0208.

Light-resistance:

The light-resistance of the polyurethane film was evaluated by irradiating the same with UV light with the use of a fadeometer at 63 ± 3 °C for 50 hours, according to JIS L 0842, and then examining the yellowing and changes in mechanical properties of the film caused thereby.

$NO_x$ resistance:

The film was exposed to $NO_x$ gas atmosphere at 23 °C, according to JIS L 0855, and the yellowing thereof thus caused was examined.

Hydrolysis-resistance:

The polyurethane film was stored at 70 °C under a relative humidity of 95 % for three weeks and then the mechanical properties thereof were measured to thereby evaluate its resistance against hydrolysis.

Example 4

To the polyurethane solution as obtained in Example 2, a hindered amine UV stabilizer, Tinuvin 144 (tradename: mfd. by Ciba-Geigy), was added in an amount of 0.5 % by weight based on the solid polyurethane. Then a polyurethane film was formed in the same manner as the one described in Examples 1 to 3 and subjected to the same performance evaluation test.

The results are shown in Table 2. Table 2 indicates that this polyurethane film exposed to photo-degradation and hydrolysis was remarkably superior to that of Example 2 in the mechanical properties.

Tinuvin 144: 2-(3,5-di-t-butyl-4-hydroxybenzyl)-2-n-butyl malonate bis(1,2,2,6,6-pentamethyl-4-piperidyl)

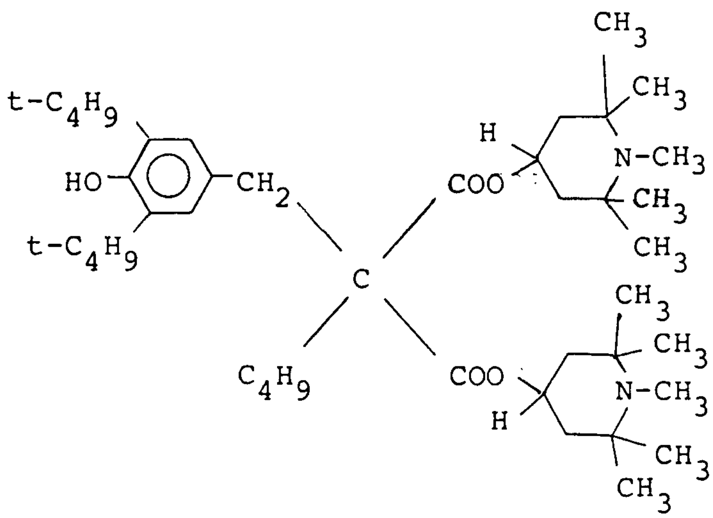

Table 1

| Prepn. Ex. No. | 1 | 2 | 3 | 4 (Comp. Ex.) |
|---|---|---|---|---|
| Polyol | A | B | C | D |
| Polyol compsn. (part by weight) ethylene glycol | 28.2 | 17.2 | 13.4 | 15.5 |
| THF | 236 | 241.4 | 194.6 | 72.7 |
| EO | 236 | 241.4 | 291.9 | 411.8 |
| $BF_3$ ethyl ether complex salt | 32.8 | 19.7 | 15.3 | 17.8 |
| Number-average MW | 1100 | 1800 | 2300 | 2000 |
| EO content (wt %) | 50 | 50 | 60 | 85 |
| Note: Number-average MW was calculated by determining OH number. EO content was calculated from the amount of the starting materials. | | | | |

Table 2

| Ex. No. | 1 | 2 | 3 | 4 |
|---|---|---|---|---|
| Polyurethane compsn. (part by wegiht) | | | | |
| polyol A | 305.7 | | | |
| polyol B | | 359.6 | 383.0 | 359.6 |
| polyol C | | | | |
| 4,4'-dicyclohexyl-methane diisocyanate | 145.6 | 104.7 | 87.3 | 104.7 |
| isophoronediamine | 48.7 | 35.7 | 29.7 | 35.7 |
| Additive: Tinuvin 144 | - | - | - | 2.5* |
| EO in polyurethane (wt %) | 31 | 36 | 46 | 36 |
| Dimensional increase at water-absorption (%) | 1.2 | 2.0 | 3.0 | 2.0 |
| Permeability ($g/m^2$ 24 hr) | 2500 | 3150 | 3600 | 3150 |
| Light resistance | | | | |
| yellowing | no | no | no | no |
| tensile strength retention (%) | - | unmeasur-able** | - | 74 |
| $NO_x$ resistance | no | no | no | no |
| Hydrolysis resistance tensile strength retention (%) | - | 7 | - | 59 |

*: 0.5 % by weight in polyurethane.

**: Highly degraded.

Comparative Example 1

370 parts by weight of the polyol D, as prepared in Preparation Example 4, containing 85 % by weight of EO units was reacted with 96.9 parts by weight of 4,4'-dicyclohexylmethane diisocyanate under the same conditions as those described in above Examples. Then the chain of the polymer thus obtained was

lengthened with 33.1 parts by weight of isophoronediamine in dimethylformamide to thereby give a colorless, transparent and viscous solution containing 25 % of solid polyurethane. The EO content in the solid polyurethane of the solution was 63 % by weight.

Subsequently a polyurethane film was formed in the same manner as those described in Examples. The obtained film was hardly available in practice since the dimensional increase thereof at water absorption was considerably high, i.e., 15 %, though its permeability was as high as 4800 g/m$^2$ 24 hr.

Comparative Example 2

286 parts by weight of polycaprolactone polyester polyol of a number-average molecular weight of 820, Placcel (tradename: mfd. by Daisel Ltd.), was reacted with 182.7 parts by weight of 4,4′-dicyclohexylmethane diisocyanate under nitrogen atmosphere at 100 ° C for six hours. Then the chain of the resulting product was lengthened with 31.3 g of 1,4-butanediol in cyclohexanone in the presence of 0.1 g of dibutyltin dilaurate at 80 ° C for six hours. Thus a colorless, transparent and viscous solution containing 25 % by weight of solid polyurethane was obtained.

With the use of this polyurethane solution, a performance evaluation test of a film was carried out in the same manner as the one described in the above Examples. As a result, the film showed a dimensional increase of almost 0 on water absorption but its permeability was extremely low, i.e., 800 g/m$^2$ 24 hr.

Examples 5 and 6

With the use of the polyol B as described above, polyurethane solutions having solid contents of 25 % by weight (Example 5) and 20 % by weight (Example 6) were prepared each according to the composition as shown in Table 3 by the same procedure as that described in Example 1. These solutions showed viscosities of 20,000 cps and 23,000 cps respectively at 25 ° C. Then polyurethane films were formed

therefrom in the same manner as the one described in Example 1 and the properties thereof were evaluated. The results are shown in Table 3 together with the data of Example 2.

Table 3

| Ex. No. | 2 | 5 | 6 |
|---|---|---|---|
| Polyurethane compsn. (part by weight) | | | |
| Polyol B | 359.6 | 330.5 | 305.8 |
| 4,4'-dicyclohexylmethane diisocyanate | 104.7 | 120.3 | 133.5 |
| isophoronediamine | 35.7 | 49.2 | 60.7 |
| EO in polyurethane (wt%) | 36 | 33 | 31 |
| Dimensional increase at water-absorption (%) | 2.0 | 1.6 | 1.3 |
| Permeability ($g/m^2$ 24 hr) | | | |
| Film thickness 10 μm | 6,700 | 6,200 | 5,500 |
| " 30 μm | 4,400 | 3,800 | 3,500 |
| " 50 μm | 3,150 | 2,800 | 2,600 |
| " 80 μm | 2,200 | 2,000 | 1,900 |
| " 100 μm | 1,900 | 1,700 | 1,600 |
| Mechanical properties | | | |
| 100% Modulus ($kg/cm^2$) | 33 | 49 | 66 |
| Tensile strength ($kg/cm^2$) | 382 | 457 | 477 |
| Elongation (%) | 676 | 640 | 606 |

## Claims

1. A nonporous film of a polyurethane resin obtained by reacting a random copolymer of tetrahydrofuran and ethylene oxide, which contains 20 to 80% by weight of ethylene oxide units and has a number-average molecular weight of 800 to 3,000, with an alicyclic diisocyanate, and lengthening the chains of the obtained product with an alicyclic diamine.

2. The nonporous film according to Claim 1, wherein the random copolymer contains 30 to 70% by weight of ethylene oxide units.

3. The nonporous film according to Claim 1, wherein the random copolymer has a number-average molecular weight of 1,000 to 2,500.

4. The nonporous film according to Claim 1, wherein the alicyclic diisocyanate is isophorone diisocyanate, 4,4′-dicyclohexylmethane diisocyanate, 1,4-cyclohexylene diisocyanate, or a hydrogenated tolylene diisocyanate.

5. The nonporous film according to Claim 4, wherein the alicyclic diisocyanate is 4,4′-dicyclohexylmethane diisocyanate.

6. The nonporous film according to Claim 1, wherein the alicyclic diamine is isophoronediamine, 4,4′-dicyclohexyldiamine, 3,3′-dimethyl-4,4′-dicyclohexylamine or 1,4-cyclohexylenediamine.

7. The nonporous film according to Claim 6, wherein the alicyclic diamine is isophoronediamine.

8. The nonporous film according to Claim 1, wherein the film has a thickness of 10 to 100 $\mu$m.

9. The nonporous film according to Claim 1, wherein the film has a permeability of at least 1,000 g/m$^2$.24 hr.

10. The nonporous film according to Claim 1, wherein the film has a 100 % modulus of at least 20 kg/cm$^2$.

11. The nonporous film according to Claim 1, wherein the film has a thickness of 10 to 100 $\mu$m, a permeability of at least 1,000 g/m$^2$.24 hr, and a 100 % modulus of at least kg/cm$^2$.

12. A medical film which comprises a nonporous film of a polyurethane resin according to claim 1 and an adhesive coated thereover.

## Revendications

1. Film non poreux en une résine de polyuréthanne obtenue par réaction entre un copolymère statistique de tétrahydrofuranne et d'oxyde d'éthylène, contenant 20 à 80 % en poids de motifs oxyde d'éthylène et ayant un poids moléculaire moyen en nombre compris entre 800 et 3 000, et un diisocyanate alicyclique, et par allongement des chaînes du produit obtenu par une diamine alicyclique.

2. Film non poreux selon la revendication 1, dans lequel le copolymère statistique contient 30 à 70 % en poids de motifs oxyde d'éthylène.

3. Film non poreux selon la revendication 1, dans lequel le copolymère statistique a un poids moléculaire moyen en nombre compris entre 1 000 et 2 500.

4. Film non poreux selon la revendication 1, dans lequel le diisocyanate alicyclique est de l'isophorone-diisocyanate, du 4,4'-dicyclohexylméthane-diisocyanate, du 1,4-cyclohexylène-diisocyanate ou un tolylène-diisocyanate hydrogéné.

5. Film non poreux selon la revendication 4, dans lequel le diisocyanate alicyclique est du 4,4'-dicyclohexylméthane-diisocyanate.

6. Film non poreux selon la revendication 1, dans lequel la diamine alicyclique est une isophoronediamine, une 4,4'-dicyclohexyldiamine, une 3,3'-diméthyl-4,4'-dicyclohexylamine ou une 1,4-cyclohexylènediamine.

7. Film non poreux selon la revendication 6, dans lequel la diamine alicyclique est une isophoronediamine.

8. Film non poreux selon la revendication 1, dans lequel le film a une épaisseur de 10 à 100 µm.

9. Film non poreux selon la revendication 1, dans lequel le film a une perméabilité supérieure ou égale à 1 000 g/m$^2$ . 24 h.

10. Film non poreux selon la revendication 1, dans lequel le film a un module de 100 % supérieur ou égal à 20 kg/cm$^2$.

11. Film non poreux selon la revendication 1, dans lequel le film a une épaisseur de 10 à 100 µm, une perméabilité supérieure ou égale à 1 000 g/m$^2$ . 24 h et un module de 100 % supérieur ou égal à 20 kg/cm$^2$.

12. Film médicinal comprenant un film non poreux en une résine de polyuréthanne selon la revendication 1, et un adhésif appliqué sur ce dernier.

**Patentansprüche**

1. Nichtporöser Film aus einem Polyurethanharz, erhalten durch Umsetzung eines statistischen Copolymerisats aus Tetrahydrofuran und Ethylenoxid, das 20 bis 80 Gew.-% Ethylenoxideinheiten enthält, und ein Zahlenmittelmolekulargewicht von 800 bis 3000 besitzt, mit einem alicyclischen Diisocyanat, und Verlängerung der Ketten des erhaltenen Produkts mit einem alicyclischen Diamin.

2. Nichtporöser Film nach Anspruch 1, wobei das statistische Copolymerisat 30 bis 70 Gew.-% Ethylenoxideinheiten enthält.

3. Nichtporöser Film nach Anspruch 1, wobei das statistische Copolymerisat ein Zahlenmittelmolekulargewicht von 1000 bis 2500 besitzt.

4. Nichtporöser Film nach Anspruch 1, wobei das alicyclische Diisocyanat Isophorondiisocyanat, 4,4'-Dicyclohexylmethandiisocyanat, 1,4-Cyclohexylendiisocyanat oder ein hydriertes Toluoldiisocyanat ist.

5. Nichtporöser Film nach Anspruch 4, wobei das alicyclische Diisocyanat 4,4'-Dicyclohexylmethandiisocyanat ist.

6. Nichtporöser Film nach Anspruch 1, wobei das alicyclische Diamin Isophorondiamin, 4,4'-Dicyclohexyldiamin, 3,3'-Dimethyl-4,4'-dicyclohexylamin oder 1,4-Cyclohexylendiamin ist.

7. Nichtporöser Film nach Anspruch 6, wobei das alicyclische Diamin Isophorondiamin ist.

8. Nichtporöser Film nach Anspruch 1, wobei der Film eine Dicke von 10 bis 100 µm besitzt.

9. Nichtporöser Film nach Anspruch 1, wobei der Film eine Permeabilität von mindestens 1000 g/m$^2$ • 24 Stunden besitzt.

10. Nichtporöser Film nach Anspruch 1, wobei der Film einen 100 % Modulus von mindestens 20 kg/cm$^2$ besitzt.

11. Nichtporöser Film nach Anspruch 1, wobei der Film eine Dicke von 10 bis 100 µm, eine Permeabilität von mindestens 1000 g/m$^2$ • 24 Stunden und einen 100 %-Modulus von mindestens 20 kg/cm$^2$ besitzt.

**12.** Medizinischer Film, umfassend einen nichtporösen Film aus einem Polyurethanharz nach Anspruch 1 und einen darauf aufgebrachten Klebstoff.